# EUROPEAN PATENT APPLICATION

(11) **EP 1 859 676 A1**
(43) Date of publication of application: **28.11.2007**
(21) Application number: 06713731.5
(22) Date of filing: 08.02.2006
(51) Int. Cl.: A01K 67/027, C12N 15/09, G01N 33/15, G01N 33/50, C12N 5/10

(54) **HAIRLESS TRANSGENIC ANIMAL**

(30) Priority: 08.02.2005 JP 2005032310
(71) Applicant: Tokyo Metropolitan Organization for Medical Research, Shinjuku-ku, Tokyo 163-8001 (JP); National University Corporation Nara Institute of Science and Technology, Ikoma-shi, Nara 630-0192 (JP)
(72) Inventor: YONEKAWA, Hiromichi, Saitama-shi, Saitama 3370007 (JP); TAKADA, Toyoyuki, Kitakatsuragi-gun, Nara 6360081 (JP); SHITARA, Hiroshi, Matsudo-shi, Chiba 2710044 (JP); KIKKAWA, Yoshiaki, Ichikawa-shi, Chiba 2720032 (JP); ISHII, Rie, Tokyo 1450064 (JP); KOHNO, K, NAT. UNIVERSITY CORP. NARA INST. SC.&TEC, Ikoma-shi, Nara 6300192 (JP)
(74) Representative: Woods, Geoffrey Corlett
(86) International application number: PCT/JP2006/302590
(87) International publication number: WO 2006/085673

(57) **Abstract**

The present invention provides a hairless transgenic nonhuman animal used in the development of a therapy for dermatitis such as human atopic dermatitis and drug discovery. Specifically, the present invention provides a transgenic nonhuman animal, into which recombinant DNA comprising a heparin-binding EGF gene and a type 2 keratin gene promoter for regulating expression of the above gene has been introduced.

SEQUENCE LISTING

<110> TOKYO METROPOLITAN ORGANIZATION FOR MEDICAL RESEARCH
NATIONAL UNIVERSITY CORPORATION NARA INSTITUTE OF SCIENCE AND
TECHNOLOGY

<120> HAIRLESS TRANSGENIC ANIMAL

<130> G07-0063

<140> PCT/JP2006/302590
<141> 2006-02-08

<150> JP 2005-32310
<151> 2005-02-08

<160> 8

<170> PatentIn version 3.3

<210> 1
<211> 35
<212> DNA
<213> Artificial

<220>
<223> primer

<400> 1
agcggccgcc agattatgaa aggaactcct gcaac 35

<210> 2
<211> 33
<212> DNA
<213> Artificial

<220>
<223> primer

<400> 2
tggatccgtt ggtagaggat ggagtaaagg tgc 33

<210> 3
<211> 29
<212> DNA
<213> Artificial

<220>
<223> primer

<400> 3
agagagcact gcggagcaac cactgaagc 29


<210> 4
<211> 28
<212> DNA
<213> Artificial

<220>
<223> primer

<400> 4
ggatcctgag aacttcaggg tgagtttg 28


<210> 5
<211> 25
<212> DNA
<213> Artificial

<220>
<223> primer

<400> 5
ccagatctgc ctcttgcaag tcacg 25


<210> 6
<211> 2360
<212> DNA
<213> Homo sapiens

<400> 6
   gctacgcggg ccacgctgct ggctggcctg acctaggcgc gcggggtcgg gcggccgcgc 60
   gggcgggctg agtgagcaag acaagacact caagaagagc gagctgcgcc tgggtcccgg 120
   ccaggcttgc acgcagaggc gggcggcaga cggtgcccgg cggaatctcc tgagctccgc 180
   cgcccagctc tggtgccagc gcccagtggc cgccgcttcg aaagtgactg gtgcctcgcc 240
   gcctcctctc ggtgcgggac catgaagctg ctgccgtcgg tggtgctgaa gctctttctg 300
   gctgcagttc tctcggcact ggtgactggc gagagcctgg agcggcttcg gagagggcta 360
   gctgctggaa ccagcaaccc ggaccctccc actgtatcca cggaccagct gctaccccta 420
   ggaggcggcc gggaccggaa agtccgtgac ttgcaagagg cagatctgga ccttttgaga 480
   gtcactttat cctccaagcc acaagcactg gccacaccaa acaaggagga gcacgggaaa 540
   agaaagaaga aaggcaaggg gctagggaag aagagggacc catgtcttcg gaaatacaag 600
   gacttctgca tccatggaga atgcaaatat gtgaaggagc tccgggctcc ctcctgcatc 660
   tgccacccgg gttaccatgg agagaggtgt catgggctga gcctcccagt ggaaaatcgc 720
   ttatatacct atgaccacac aaccatcctg gccgtggtgg ctgtggtgct gtcatctgtc 780
   tgtctgctgg tcatcgtggg gcttctcatg tttaggtacc ataggagagg aggttatgat 840
   gtggaaaatg aagagaaagt gaagttgggc atgactaatt cccactgaga gagacttgtg 900
   ctcaaggaat cggctgggga ctgctacctc tgagaagaca caaggtgatt tcagactgca 960
   gaggggaaag acttccatct agtcacaaag actccttcgt ccccagttgc cgtctaggat 1020
   tgggcctccc ataattgctt tgccaaaata ccagagcctt caagtgccaa acagagtatg 1080
   tccgatggta tctgggtaag aagaaagcaa aagcaaggga ccttcatgcc cttctgattc 1140
   ccctccacca aaccccactt cccctcataa gtttgtttaa acacttatct tctggattag 1200
   aatgccggtt aaattccata tgctccagga tctttgactg aaaaaaaaaa agaagaagaa 1260
   gaaggagagc aagaaggaaa gatttgtgaa ctggaagaaa gcaacaaaga ttgagaagcc 1320
   atgtactcaa gtaccaccaa gggatctgcc attgggaccc tccagtgctg gatttgatga 1380
   gttaactgtg aaataccaca agcctgagaa ctgaattttg ggacttctac ccagatggaa 1440
   aaataacaac tatttttgtt gttgttgttt gtaaatgcct cttaaattat atatttattt 1500
   tattctatgt atgttaattt atttagtttt taacaatcta acaataatat ttcaagtgcc 1560
   tagactgtta ctttggcaat ttcctggccc tccactcctc atccccacaa tctggcttag 1620
   tgccacccac ctttgccaca aagctaggat ggttctgtga cccatctgta gtaatttatt 1680
   gtctgtctac atttctgcag atcttccgtg gtcagagtgc cactgcggga gctctgtatg 1740
   gtcaggatgt aggggttaac ttggtcagag ccactctatg agttggactt cagtcttgcc 1800
   taggcgattt tgtctaccat ttgtgttttg aaagcccaag gtgctgatgt caaagtgtaa 1860
   cagatatcag tgtctccccg tgtcctctcc ctgccaagtc tcagaagagg ttgggcttcc 1920
   atgcctgtag ctttcctggt ccctcacccc catggcccca ggccacagcg tgggaactca 1980
   ctttcccttg tgtcaagaca tttctctaac tcctgccatt cttctggtgc tactccatgc 2040
   aggggtcagt gcagcagagg acagtctgga gaaggtatta gcaaagcaaa aggctgagaa 2100
   ggaacaggga acattggagc tgactgttct tggtaactga ttacctgcca attgctaccg 2160
   agaaggttgg aggtggggaa ggctttgtat aatcccaccc acctcaccaa aacgatgaag 2220
   gtatgctgtc atggtccttt ctggaagttt ctggtgccat ttctgaactg ttacaacttg 2280
   tatttccaaa cctggttcat atttatactt tgcaatccaa ataaagataa cccttattcc 2340
   ataaaaaaaa aaaaaaaaaa 2360

<210> 7

<211> 8608
<212> DNA
<213> Mus musclus

<400> 7
ccagattatg aaaggaactc ctgcaacttc acaggacaag gacagcctag ttaagaaagg 60

tgcaaagggg ccagagagat ggttcagcag ttaagagcac tgcctgctct tccaggagac 120

ccaaattcaa tttccagtgt gcacatcagg tggttcacaa ccacctgtaa ttccagctcc 180

agagggagac caacatccct ggcttcttcg tacatctgca tccacatgca caacccttcc 240

cacaattaaa attgatatat tttttaaaaa atagaagtgt gcaaaggaca ttagtggatt 300

ttcacctaaa gaaagtatat gtatggtcaa tgaacacatg aaagtatgtt caacataatt 360

agactttatg aaaaggggga ccaaacccac aatttatatc ttataagaca atttaatatt 420

aaaaacatta atatagagag ggaaataaca gatactggtg aggatgaaga gaagctgaaa 480

tttttgtgct tttggttttg ttttgagata ggatttcatt gtgtagcccc gtttggcctg 540

gagctcactc tgtagaccag gctggcctca tggtcttaaa gatccacttg cttctgcctc 600

ccaagtactg ggcttaaggg tatgtgctac taagcccggt gctactaagg ttagaatctt 660

tgtatggtcc tggtgggaat ggattgttgg ctcagcagtt aagagcactt gggacccttg 720

cagaagatgc aggtttggtt cctactcccc acacatggtg gttctcaatc atctcccatt 780

ctaggggatc ccgtgccttc ttctgatgtc agtaggcact aggcatgtat gtggtacaca 840

tacatacaca caagtacaca tacatacaac ataataaaat aaatataagg attatcatgt 900

gccagtaagt ccactcctag aaatacaggc aaaagaactg aaatcagata ttccaacagt 960

acatgtaaca tagatgctca gaactgtact atacaaaaca gcccaaacat ctgtcagtga 1020

gtgaatagaa agagacagac agacagagat agggacagaa acaggcagag acagagacac 1080

agtgagatac agagagacat aaatacagac tgagacacac agagagacag agaaagggac 1140

agagatagtc cgagacagag agagactcag agagaaggag agggagaggg agagggagag 1200

ggagagagag agagagagag aaagagagag agagagggag agagagagag agttttcccc 1260

attgcatgac ctattcatag aaagtatcca gattaggaaa tccaaatatt cagaaacagg 1320

gggaatagca gattggtggc aatagatgca gggctttctt tggggtgatg aaatgttttg 1380

gaactaggca gatctggttg tctataccac tgggaacata gaaatgtcac ttaactgttc 1440

actaaagtgg ttagttttat ggtctataaa tttcatttca ataaaactaa aattgaatgt 1500

gagagtgtta aactaaagag gccataaaaa cggtatccct agacttaaat tcccagtcct 1560

tccatgattc atcaaatggt atggcaaggg gaactgtcca gggctaatag aacacaggga 1620

caacatactc tctattcagc tggctttgag cagggcaaac tcatctgtga tggccgagag 1680

ataaataact gggacactgg cttccacaca atgaggcagc tgtaggtttt cttacctggt 1740

catctgatta tgtaactagc tacctgggtc tcaagcttgg catggtggac agctggtagc 1800

ccagtgcctg tgtttgcctt ggcgagctaa agcaatggaa gctgacaaat tacaacacat 1860

ctttctcagc cagaactgtg gcccctgccc tggagcttgg atcaggatgt gtgtgtgtgt 1920

gtgtgtgtgt gtgtgtgtgt gtccacacct gtgtacgtgt tcacatgtgt atctgtgtat 1980

ataggtaggc tatggttcaa cttcagatat ctttgtctat tgctctccac tctatatcta 2040

tctatctatc tatctatcta tctatctatc tatctatcta tctatctatc tatctatcta 2100

tcatctacct atctataggt tctctccttg aacttggagc tcatcagcca gatttagcta 2160

ctctgattgg ccagcgagcc cctgggatcc tcctatctgt gcctcccagt gctagaattg 2220

caggcatctc ccaccaggcc tggtttctat gcaggtgctc agcatctgag ctccgttgct 2280

cacattttta tggtaagcac tttgaccact ggagttacct tcctaacctt ttagatcagg 2340

tatttaagat agaatcttgc ttctctccat tcaggattca gcagaagaga tcaagcttta 2400

ctgggccgag aagtcctcta aggcaggctg tcttccttta tcactgtatg gtagatgcct 2460

ggcatagaga ggcagcttga caaatgctga ctgagtcatt tgggaaggga ccatcctctc 2520

agccctctcc tgcttccata gtctcggggt tgggtggaag tcgtgaatga ctacctcacc 2580

ctgtcagcag ctggagtcat ggcctccgtt tgtgggctat cattgagcaa gaatactttg 2640

gagttagaga cagaagcact ctgggacaga gggacaggat ccaaacaact tggtatttac 2700

ttaccactac tggtcaagag ttagacagac aagaagaagc agaaaccaga ggctctggga 2760

ggtgagagtt gagtgtgtgc acagaaagcc aggctatgag aggcccaggt cacaggctat 2820

ggccactcta ccagcagcca agctatggtg cagttcctca cctctgtgcc acctcatgga 2880

aattgccaaa tttcctttgg cattaatgtc tcccttaaga agcagggata ataatagtgc 2940

cagccttcag ggctgttgtg aggattagat gaattaatat atgtgaggtg cttagaacca 3000

cggctggcac gatgaaccct gactaagtgt tagctagccc agtcacggtc accttcctta 3060

ttactattta cagggtatct tcttccatcc accttgggga gtaggtggaa cggatgttgg 3120

tgtctcctca ttagggatgt agagaaggta gaagccctac ccactgttac ataggtcatg 3180

agtggctcag gagagactat gacatggatg cttaagagtc ccaagtccac acactatgcc 3240

agaagtgtgg agtcagcagg taaagaagag atgctcaacc atgcccaagg gaagtatcct 3300

ggttggaggt gacaggggac agaactggcc cgaatgacat ctcacaaagc aaaacccgca 3360

ctgtggctgg gtgggagcag agctgcgttc agtacatgtc cctgtccttt atgcccagct 3420

ccttgggggt gagctcacct ttggcctttt aagtactaaa cctgctgagc acctagggac 3480

tgcagtcagt ccagctgact caggctgagg gcactgccta cctgaggcca tgcaagcctg 3540

caaggagacc ctgtttgggg aattcacaga cagcacactc atgaccttct ccatcagccg 3600

gggacgcctg caaggacatg agctgatgtc attcaaatag cccagctcat tttctctaat 3660

cttggagata attactttgc tcagaaaact acccggccct ctcctggtgc ctctcacctc 3720

tcccactccc aattctaccc acagaggaac agattttgcc tcagatgttc agtttaggtt 3780

tcttcagact caacagtctt cagtagctcc ctgtcttaca cacagcttgg gttctggaac 3840

ttctctgccc aaggtttccc tctcctcaca atcacttcag cctactcagt ggcctcattt 3900

cctcacaaat gacctgtgtc tcaccatcct gggcggcttc cctgatttat tttatgtaac 3960

tacatttcaa atatcatccc cttttcctgg tttcccctct gcaaaccccc tatcccatcc 4020

ccccttatcc tgcttctatg agggtgctcc gccacccatc cacccactcc tgcctctcca 4080

ccctagcatt cctctacact ggggcatcaa gccttcatgg gaccaagggt ctcccctccc 4140

attggtgcta gataaggccc cttcagctcc ttcagtcctt tcccaactct tccattgggg 4200

tccctgtgct cagcctgata gttggctgca agcctctgca tctgtattgg ccaggatctg 4260

gcagagcctc tcaggagaca gtggtatcag gctcctgtca gcaagcactt cttggcatct 4320

gcaatagtgt ctaggtttgg tgtctgcata tggatggatc cctaggtggg aacactctct 4380

gggtggcctt tccattaatc tctgctctac tctttgtccc tgtatttcct ttagacagga 4440

gcaattctgg gttaaaattt tggagatggg tgggtggccc tagccctcaa ccagggggcc 4500

atcctaacct ctggatatgg ttccctgact tcttagaacg gtcaattcaa tgcttcctcc 4560

cttaaaggat tggaatccaa gcctctcctg caggctgctt gcgtgtttag aatccaagcc 4620

tctcctgcag gcggcctgtg tgcttagaat ccaagcctct cctgcaggct gcctgtgtgc 4680

ttagaatcca agcctctcct gcaggctgcc tgtgtgctta gaatccaagc ctctcctgca 4740

ggctgcctgt gtgctcttag tttctcaagg tcacatcttt gctcatttcc ttctccaacc 4800

ccgaaactgc agtctcatgg gctcaagtct gtcctagaga ttttctctca agaggcttat 4860

tggcccaaga cctctgcaaa gggacagcag atgcaccaaa caaaaccatg gaacacaaac 4920

aaacgtgtca actgattgtt gccacactca gaagcacaga tgcaaagaaa actacaattt 4980

aaataaaaga actaagtaca cagccaagag ccataccacg gaagatcctt gggaccctca 5040

tcacgctcag aaaacaccat ttctctcatg tagacatcat gaacagaaga ccagagtgca 5100

gaggagacag gccaggccag aagccagggt ttcagcaatt tttcagagaa gagggatatg 5160

ggccaagttg attagataag ccactttcct ttcccttgaa tgtccaagcc agatcttcct 5220

ggtatcttca gggttttatt ctatatctat ctccatcacc tctctatttc acaggtgagc 5280

aacgctgtct ccctaagaca tgtgcggata ggtgagcatc actaaaagtc agtttttttt 5340

ttacagagag ccacctgtgt agggacaggt gggtaaggtc tccatcatca ggtcatggga 5400

ttcccaaagc atggggtctg ccttctcctg cccacaattc tctccagatc tcaactagga 5460

cacaggcaaa ggcagaggcc tttgtgaact ggggctccat ttggtctttt ttttttttaa 5520

tccctccctt taccctctag caaaatgtaa gcatcccagg tacccgatct gggcccaccc 5580

ttcctgtggg tggaggtaag cgctttggag ggcaacatgc tttggattgt tccgatcaga 5640

atttggttct gtgtccaaca aataggggat aaatgggcca gcagaaatag atgtgacata 5700

atgtctttaa agtgcttcct actgagtttt atacctggca gccacttggt tgagtgtggc 5760

tatcactact attattattc gtaatggtca attttctggg atcggcagca gtctaggaga 5820

gcctggggca gatagggtca ccaccccttc cctgtcctca gcatctcatg ccaccgtgtg 5880

ggaatgtgct gatgaaggtg ccatgctgga gaggaaggag ggaagacagg ccgagcctct 5940

ggctctttag tcaccaacac cggttattgg acattaagaa tgtgtcaggc cttagggagt 6000

gcttcctgtg gaataacacg aacagacctt gacgttcctg ggttaatgtc tattgttaca 6060

ttctctgtgg gagaagctga gaccagaaag gccaaactga gtggtggaga agaagttgat 6120

tccctggggt ccaggctacc atctggtgcc actccaggtg atcaaaagtc cagcatccat 6180

ctcggctctt taccgtgtct ccagtgaggg actgcctcct gggagggtta gggttagtag 6240

gtccagacct gcaaggggcc cacagaagga ggcatcactg gctcctggac cttcatcatg 6300

ctcagaaaac accatttctc tcatgtagat gtcatgaata gaagaccaga gtgcagagga 6360

gacaggcgag gtcggaagcc agggtttagg cagaattcag agaagaggga tgtgggacag 6420

aggtggtgac agaagtagtc aggggtgggt caggatagct taggtaaaca tgagggggca 6480

ctaagatgag gccagaggac gaggtgtcca tgggcagaca gggagcagta agcaacagtg 6540

ggtgggtagg agcagaggat gcagggcaca gctgcctggg caggggtgct gccatgtatg 6600

tcagaggact gtgttgctgg caaaaccctt gacaggggtt ctgtgccagg gctatgctct 6660

gagagcgcag aaaaagcccg tcagcatcac acagacattc tgtgggaact gagtggcggc 6720

taacagggct gggggagtag gagaggggag gggtgcggag gggtggtagg agagggccat 6780

gctaggcgtc tttggtctgg agaagctctg ctatactcac aaaggaagct aagatggtgc 6840

atggtgtgga tgcgggagga gcagcaagga agttgttacc atgtatccaa gagcatgtcc 6900

aggacagaga gaggggggcg gagctcagac ccagctatag tggtctagga ggatctgtga 6960

ggagggctgg gtacagtgtg agagaataaa agataggaga ggtgggtgaa ggatggatgc 7020

agagaggggg agaagagggt ggagggagca agctataaca ttttgatgtc agtgatgaag 7080

acaagacagc aggaaagaat aggaagggat acagccaagg agagagtagg cttcgtgccc 7140

actgaatcca gacagagccc tagcccagcc catgtccaga tttttctccc tttgaaatcc 7200

tactcagatg agagccccac ctcactatgg ggccttgtca gtgacttttg agggaggcca 7260

ttctccagga gtgagacagc catggcctgg acagaatgat taatggaggg actggggccc 7320

atcatgggag cctccgacct gggatagagg ggaccacaga cagggagcac ataccaaaaa 7380

accgaagaat acaacataaa acctgatgtg gtgtacagct atattcccat tgagggcttt 7440

ctggcattag ctgtgtgaaa tctagatgcc taggaatggc cccagccagg ttggactccc 7500

cagttcccag gcctgggcag tagtagctgg agggagggcc catgggtgac aattgattcc 7560

tgcccttact gtgaacatgc agatgaacat ggcaggagac aggcatgatg aattggaggt 7620

ttcgtggggc acctttgaac tccccagacc gtgtggtcct ctgtgctttc tgcccatgga 7680

gtccccacca aactccactc tcttgccttt tcattttccc caagagccag aggcttgtcc 7740

tttccttcag tctcttaccg gctttattta ctatggggtt gacagctgtg ctgggcattg 7800

tctacctctc tggaaagtgt gataagcaca tggctgtcat ttagcccaca gtcgttgtga 7860

cacaggtccc atgataaaaa tagggtcctg tcatcctggt accccaaaag gagagaccca 7920

cttctctcca tggcttatat ctgcaggagg cctggtgaga aagtctgctg gagaatggcc 7980

tccctcaaag gtctctgaca aggccccata gtgagatagt gttttcagct gagtaggatt 8040

ctaaagcaag aaaaatctgg acacaggctg ggttagggct ctatctggat atagctagtc 8100

tcaattgggc ttcccctttc taggacaggg gatacagctg tggtagagtc cttgggcatt 8160

gtacatagtg gcctgctctg tccaaaatgt ctacgccatc tctgcctctg aacccctgta 8220

agaaagccag ccgcagagat gaatctggga gatggtgaag tcctggtgac attcccctga 8280

ggaggctgtg gcctggggca aattacctta taacagcggg ttaattagat aagacccaaa 8340

ttaccagagc aaagtttgca agcccagcaa tttaacagtt tcatgttgtt tcagaggtgg 8400

ggagggccga tctctggctc agcacagaga aaatgacttc ttgctcccag gcaagccaaa 8460

cccacgaatt aagctctcca ctgcccatct gctagcccaa tgccaagcat atataaaggc 8520

acctgctggt cctcactgca gccagcacaa ccttcgtggg aatctacctt cctcctgcac 8580

ctttactcca tcctctacca acggatcc 8608

<210> 8
<211> 11038
<212> DNA
<213> Artificial

<220>
<223> vector

<400> 8
ccagattatg aaaggaactc ctgcaacttc acaggacaag gacagcctag ttaagaaagg 60

tgcaaagggg ccagagagat ggttcagcag ttaagagcac tgcctgctct tccaggagac 120

ccaaattcaa tttccagtgt gcacatcagg tggttcacaa ccacctgtaa ttccagctcc 180

agagggagac caacatccct ggcttcttcg tacatctgca tccacatgca caacccttcc 240

cacaattaaa attgatatat tttttaaaaa atagaagtgt gcaaaggaca ttagtggatt 300

ttcacctaaa gaaagtatat gtatggtcaa tgaacacatg aaagtatgtt caacataatt 360

agactttatg aaaaggggga ccaaacccac aatttatatc ttataagaca atttaatatt 420

aaaaacatta atatagagag ggaaataaca gatactggtg aggatgaaga gaagctgaaa 480

tttttgtgct tttggttttg ttttgagata ggatttcatt gtgtagcccc gtttggcctg 540

gagctcactc tgtagaccag gctggcctca tggtcttaaa gatccacttg cttctgcctc 600

ccaagtactg ggcttaaggg tatgtgctac taagcccggt gctactaagg ttagaatctt 660

tgtatggtcc tggtgggaat ggattgttgg ctcagcagtt aagagcactt gggacccttg 720

cagaagatgc aggtttggtt cctactcccc acacatggtg gttctcaatc atctcccatt 780

ctaggggatc ccgtgccttc ttctgatgtc agtaggcact aggcatgtat gtggtacaca 840

tacatacaca caagtacaca tacatacaac ataataaaat aaatataagg attatcatgt 900

gccagtaagt ccactcctag aaatacaggc aaaagaactg aaatcagata ttccaacagt 960

acatgtaaca tagatgctca gaactgtact atacaaaaca gcccaaacat ctgtcagtga 1020

gtgaatagaa agagacagac agacagagat agggacagaa acaggcagag acagagacac 1080

agtgagatac agagagacat aaatacagac tgagacacac agagagacag agaaagggac 1140

agagatagtc cgagacagag agagactcag agagaaggag agggagaggg agagggagag 1200

ggagagagag agagagagag aaagagagag agagagggag agagagagag agttttcccc 1260

attgcatgac ctattcatag aaagtatcca gattaggaaa tccaaatatt cagaaacagg 1320

gggaatagca gattggtggc aatagatgca gggctttctt tggggtgatg aaatgttttg 1380

gaactaggca gatctggttg tctataccac tgggaacata gaaatgtcac ttaactgttc 1440

actaaagtgg ttagttttat ggtctataaa tttcatttca ataaaactaa aattgaatgt 1500

gagagtgtta aactaaagag gccataaaaa cggtatccct agacttaaat tcccagtcct 1560

tccatgattc atcaaatggt atggcaaggg gaactgtcca gggctaatag aacacaggga 1620

caacatactc tctattcagc tggctttgag cagggcaaac tcatctgtga tggccgagag 1680

ataaataact gggacactgg cttccacaca atgaggcagc tgtaggtttt cttacctggt 1740

catctgatta tgtaactagc tacctgggtc tcaagcttgg catggtggac agctggtagc 1800

ccagtgcctg tgtttgcctt ggcgagctaa agcaatggaa gctgacaaat tacaacacat 1860

ctttctcagc cagaactgtg gcccctgccc tggagcttgg atcaggatgt gtgtgtgtgt 1920

gtgtgtgtgt gtgtgtgtgt gtccacacct gtgtacgtgt tcacatgtgt atctgtgtat 1980

ataggtaggc tatggttcaa cttcagatat ctttgtctat tgctctccac tctatatcta 2040

tctatctatc tatctatcta tctatctatc tatctatcta tctatctatc tatctatcta 2100

tcatctacct atctataggt tctctccttg aacttggagc tcatcagcca gatttagcta 2160

ctctgattgg ccagcgagcc cctgggatcc tcctatctgt gcctcccagt gctagaattg 2220

caggcatctc ccaccaggcc tggtttctat gcaggtgctc agcatctgag ctccgttgct 2280

cacattttta tggtaagcac tttgaccact ggagttacct tcctaacctt ttagatcagg 2340

tatttaagat agaatcttgc ttctctccat tcaggattca gcagaagaga tcaagcttta 2400

ctgggccgag aagtcctcta aggcaggctg tcttccttta tcactgtatg gtagatgcct 2460

ggcatagaga ggcagcttga caaatgctga ctgagtcatt tgggaaggga ccatcctctc 2520

agccctctcc tgcttccata gtctcggggt tgggtggaag tcgtgaatga ctacctcacc 2580

ctgtcagcag ctggagtcat ggcctccgtt tgtgggctat cattgagcaa gaatactttg 2640

gagttagaga cagaagcact ctgggacaga gggacaggat ccaaacaact tggtatttac 2700

ttaccactac tggtcaagag ttagacagac aagaagaagc agaaaccaga ggctctggga 2760

ggtgagagtt gagtgtgtgc acagaaagcc aggctatgag aggcccaggt cacaggctat 2820

ggccactcta ccagcagcca agctatggtg cagttcctca cctctgtgcc acctcatgga 2880

aattgccaaa tttcctttgg cattaatgtc tcccttaaga agcagggata ataatagtgc 2940

cagccttcag ggctgttgtg aggattagat gaattaatat atgtgaggtg cttagaacca 3000

cggctggcac gatgaaccct gactaagtgt tagctagccc agtcacggtc accttcctta 3060

ttactattta cagggtatct tcttccatcc accttgggga gtaggtggaa cggatgttgg 3120

tgtctcctca ttagggatgt agagaaggta gaagccctac ccactgttac ataggtcatg 3180

agtggctcag gagagactat gacatggatg cttaagagtc ccaagtccac acactatgcc 3240

agaagtgtgg agtcagcagg taaagaagag atgctcaacc atgcccaagg gaagtatcct 3300

ggttggaggt gacaggggac agaactggcc cgaatgacat ctcacaaagc aaaacccgca 3360

ctgtggctgg gtgggagcag agctgcgttc agtacatgtc cctgtccttt atgcccagct 3420

ccttgggggt gagctcacct ttggcctttt aagtactaaa cctgctgagc acctagggac 3480

tgcagtcagt ccagctgact caggctgagg gcactgccta cctgaggcca tgcaagcctg 3540

caaggagacc ctgtttgggg aattcacaga cagcacactc atgaccttct ccatcagccg 3600

gggacgcctg caaggacatg agctgatgtc attcaaatag cccagctcat tttctctaat 3660

cttggagata attactttgc tcagaaaact acccggccct ctcctggtgc ctctcacctc 3720

tcccactccc aattctaccc acagaggaac agattttgcc tcagatgttc agtttaggtt 3780

tcttcagact caacagtctt cagtagctcc ctgtcttaca cacagcttgg gttctggaac 3840

ttctctgccc aaggtttccc tctcctcaca atcacttcag cctactcagt ggcctcattt 3900

cctcacaaat gacctgtgtc tcaccatcct gggcggcttc cctgatttat tttatgtaac 3960

tacatttcaa atatcatccc cttttcctgg tttcccctct gcaaaccccc tatcccatcc 4020

ccccttatcc tgcttctatg agggtgctcc gccacccatc cacccactcc tgcctctcca 4080

ccctagcatt cctctacact ggggcatcaa gccttcatgg gaccaagggt ctcccctccc 4140

attggtgcta gataaggccc cttcagctcc ttcagtcctt tcccaactct tccattgggg 4200

tccctgtgct cagcctgata gttggctgca agcctctgca tctgtattgg ccaggatctg 4260

gcagagcctc tcaggagaca gtggtatcag gctcctgtca gcaagcactt cttggcatct 4320

gcaatagtgt ctaggtttgg tgtctgcata tggatggatc cctaggtggg aacactctct 4380

gggtggcctt tccattaatc tctgctctac tctttgtccc tgtatttcct ttagacagga 4440

gcaattctgg gttaaaattt tggagatggg tgggtggccc tagccctcaa ccagggggcc 4500

atcctaacct ctggatatgg ttccctgact tcttagaacg gtcaattcaa tgcttcctcc 4560

cttaaaggat tggaatccaa gcctctcctg caggctgctt gcgtgtttag aatccaagcc 4620

tctcctgcag gcggcctgtg tgcttagaat ccaagcctct cctgcaggct gcctgtgtgc 4680

ttagaatcca agcctctcct gcaggctgcc tgtgtgctta gaatccaagc ctctcctgca 4740

ggctgcctgt gtgctcttag tttctcaagg tcacatcttt gctcatttcc ttctccaacc 4800

ccgaaactgc agtctcatgg gctcaagtct gtcctagaga ttttctctca agaggcttat 4860

tggcccaaga cctctgcaaa gggacagcag atgcaccaaa caaaaccatg gaacacaaac 4920

aaacgtgtca actgattgtt gccacactca gaagcacaga tgcaaagaaa actacaattt 4980

aaataaaaga actaagtaca cagccaagag ccataccacg gaagatcctt gggaccctca 5040

tcacgctcag aaaacaccat ttctctcatg tagacatcat gaacagaaga ccagagtgca 5100

gaggagacag gccaggccag aagccagggt ttcagcaatt tttcagagaa gagggatatg 5160

ggccaagttg attagataag ccactttcct ttcccttgaa tgtccaagcc agatcttcct 5220

ggtatcttca gggttttatt ctatatctat ctccatcacc tctctatttc acaggtgagc 5280

aacgctgtct ccctaagaca tgtgcggata ggtgagcatc actaaaagtc agtttttttt 5340

ttacagagag ccacctgtgt agggacaggt gggtaaggtc tccatcatca ggtcatggga 5400

ttcccaaagc atggggtctg ccttctcctg cccacaattc tctccagatc tcaactagga 5460

cacaggcaaa ggcagaggcc tttgtgaact ggggctccat ttggtctttt ttttttttaa 5520

tccctccctt taccctctag caaaatgtaa gcatcccagg tacccgatct gggcccaccc 5580

ttcctgtggg tggaggtaag cgctttggag ggcaacatgc tttggattgt tccgatcaga 5640

atttggttct gtgtccaaca aataggggat aaatgggcca gcagaaatag atgtgacata 5700

atgtctttaa agtgcttcct actgagtttt atacctggca gccacttggt tgagtgtggc 5760

tatcactact attattattc gtaatggtca attttctggg atcggcagca gtctaggaga 5820

gcctggggca gatagggtca ccaccccttc cctgtcctca gcatctcatg ccaccgtgtg 5880

ggaatgtgct gatgaaggtg ccatgctgga gaggaaggag ggaagacagg ccgagcctct 5940

ggctctttag tcaccaacac cggttattgg acattaagaa tgtgtcaggc cttagggagt 6000

gcttcctgtg gaataacacg aacagacctt gacgttcctg ggttaatgtc tattgttaca 6060

ttctctgtgg gagaagctga gaccagaaag gccaaactga gtggtggaga agaagttgat 6120

tccctggggt ccaggctacc atctggtgcc actccaggtg atcaaaagtc cagcatccat 6180

ctcggctctt taccgtgtct ccagtgaggg actgcctcct gggagggtta gggttagtag 6240

gtccagacct gcaaggggcc cacagaagga ggcatcactg gctcctggac cttcatcatg 6300

ctcagaaaac accatttctc tcatgtagat gtcatgaata gaagaccaga gtgcagagga 6360

gacaggcgag gtcggaagcc agggtttagg cagaattcag agaagaggga tgtgggacag 6420

aggtggtgac agaagtagtc aggggtgggt caggatagct taggtaaaca tgagggggca 6480

ctaagatgag gccagaggac gaggtgtcca tgggcagaca gggagcagta agcaacagtg 6540

ggtgggtagg agcagaggat gcagggcaca gctgcctggg caggggtgct gccatgtatg 6600

tcagaggact gtgttgctgg caaaaccctt gacaggggtt ctgtgccagg gctatgctct 6660

gagagcgcag aaaaagcccg tcagcatcac acagacattc tgtgggaact gagtggcggc 6720

taacagggct gggggagtag gagaggggag gggtgcggag gggtggtagg agagggccat 6780

gctaggcgtc tttggtctgg agaagctctg ctatactcac aaaggaagct aagatggtgc 6840

atggtgtgga tgcgggagga gcagcaagga agttgttacc atgtatccaa gagcatgtcc 6900

aggacagaga gaggggggcg gagctcagac ccagctatag tggtctagga ggatctgtga 6960

ggagggctgg gtacagtgtg agagaataaa agataggaga ggtgggtgaa ggatggatgc 7020

agagaggggg agaagagggt ggagggagca agctataaca ttttgatgtc agtgatgaag 7080

acaagacagc aggaaagaat aggaagggat acagccaagg agagagtagg cttcgtgccc 7140

actgaatcca gacagagccc tagcccagcc catgtccaga tttttctccc tttgaaatcc 7200

tactcagatg agagccccac ctcactatgg ggccttgtca gtgacttttg agggaggcca 7260

ttctccagga gtgagacagc catggcctgg acagaatgat taatggaggg actggggccc 7320

atcatgggag cctccgacct gggatagagg ggaccacaga cagggagcac ataccaaaaa 7380

accgaagaat acaacataaa acctgatgtg gtgtacagct atattcccat tgagggcttt 7440

ctggcattag ctgtgtgaaa tctagatgcc taggaatggc cccagccagg ttggactccc 7500

cagttcccag gcctgggcag tagtagctgg agggagggcc catgggtgac aattgattcc 7560

tgcccttact gtgaacatgc agatgaacat ggcaggagac aggcatgatg aattggaggt 7620

ttcgtggggc acctttgaac tccccagacc gtgtggtcct ctgtgctttc tgcccatgga 7680

gtccccacca aactccactc tcttgccttt tcattttccc caagagccag aggcttgtcc 7740

tttccttcag tctcttaccg gctttattta ctatggggtt gacagctgtg ctgggcattg 7800

tctacctctc tggaaagtgt gataagcaca tggctgtcat ttagcccaca gtcgttgtga 7860

cacaggtccc atgataaaaa tagggtcctg tcatcctggt accccaaaag gagagaccca 7920

cttctctcca tggcttatat ctgcaggagg cctggtgaga aagtctgctg gagaatggcc 7980

tccctcaaag gtctctgaca aggccccata gtgagatagt gttttcagct gagtaggatt 8040

ctaaagcaag aaaaatctgg acacaggctg ggttagggct ctatctggat atagctagtc 8100

tcaattgggc ttcccctttc taggacaggg gatacagctg tggtagagtc cttgggcatt 8160

gtacatagtg gcctgctctg tccaaaatgt ctacgccatc tctgcctctg aacccctgta 8220

agaaagccag ccgcagagat gaatctggga gatggtgaag tcctggtgac attcccctga 8280

ggaggctgtg gcctggggca aattacctta taacagcggg ttaattagat aagacccaaa 8340

ttaccagagc aaagtttgca agcccagcaa tttaacagtt tcatgttgtt tcagaggtgg 8400

ggagggccga tctctggctc agcacagaga aaatgacttc ttgctcccag gcaagccaaa 8460

cccacgaatt aagctctcca ctgcccatct gctagcccaa tgccaagcat atataaaggc 8520

acctgctggt cctcactgca gccagcacaa ccttcgtggg aatctacctt cctcctgcac 8580

ctttactcca tcctctacca acggatcctg agaacttcag ggtgagtttg gggacccttg 8640

attgttcttt ctttttcgct attgtaaaat tcatgttata tggagggggc aaagttttca 8700

gggtgttgtt tagaatggga agatgtccct tgtatcacca tggaccctca tgataatttt 8760

gtttctttca ctttctactc tgttgacaac cattgtctcc tcttattttc ttttcatttt 8820

ctgtaacttt ttcgttaaac tttagcttgc atttgtaacg aatttttaaa ttcacttttg 8880

tttatttgtc agattgtaag tactttctct aatcactttt ttttcaaggc aatcagggta 8940

tattatattg tacttcagca cagttttaga gaacaattgt tataattaaa tgataaggta 9000

gaatatttct gcatataaat tctggctggc gtggaaatat tcttattggt agaaacaact 9060

acaccctggt catcatcctg cctttctctt tatggttaca atgatataca ctgtttgaga 9120

tgaggataaa atactctgag tccaaaccgg gcccctctgc taaccatgtt catgccttct 9180

tctctttcct acagctcctg ggcaacgtgc tggttgttgt gctgtctcat cattttggca 9240

aagaattccg atccactagt aacggccgcc agtgtgctct aaagcgcgta tcgaaagtga 9300

ctggtgcctc gccgcctcct ctcggtgcgg gaccatgaag ctgctgccgt cggtggtgct 9360

gaagctcttt ctggctgcag ttctctcggc actggtgact ggcgagagcc tggagcggct 9420

tcggagaggg ctagctgctg gaaccagcaa cccggaccct cccactgtat ccacggacca 9480

gctgctaccc ctaggaggcg gccgggaccg gaaagtccgt gacttgcaag aggcagatct 9540

ggaccttttg agagtcactt tatcctccaa gccacaagca ctggccacac caaacaagga 9600

ggagcacggg aaaagaaaga agaaaggcaa ggggctaggg aagaagaggg acccatgtct 9660

tcggaaatac aaggacttct gcatccatgg agaatgcaaa tatgtgaagg agctccgggc 9720

tccctcctgc atctgccacc cgggttacca tggagagagg tgtcatgggc tgagcctccc 9780

agtggaaaat cgcttatata cctatgacca cacaaccatc ctggccgtgg tggctgtggt 9840

gctgtcatct gtctgtctgc tggtcatcgt ggggcttctc atgtttaggt accataggag 9900

aggaggttat gatgtggaaa atgaagagaa agtgaagttg ggcatgacta attcccactg 9960

agagagactt gtgctcaagg aatcggctgg ggactgctac ctctgagaag acacaaggtg 10020

atttcagact gcaggtcgac tttagagcac actggcggcc ggaattcact cctcaggtgc 10080

aggctgccta tcagaaggtg gtggctggtg tggccaatgc cctggctcac aaataccact 10140

gagatctttt tccctctgcc aaaaattatg gggacatcat gaagcccctt gagcatctga 10200

cttctggcta ataaaggaaa tttattttca ttgcaatagt gtgttggaat tttttgtgtc 10260

tctcactcgg aaggacatat gggagggcaa atcatttaaa acatcagaat gagtatttgg 10320

tttagagttt ggcaacatat gccatatgct ggctgccatg aacaaaggtg gctataaaga 10380

ggtcatcagt atatgaaaca gccccctgct gtccattcct tattccatag aaaagccttg 10440

acttgaggtt agattttttt tatattttgt tttgtgttat ttttttcttt aacatcccta 10500

aaattttcct tacatgtttt actagccaga tttttcctcc tctcctgact actcccagtc 10560

atagctgtcc ctcttctctt atgaagatct tattaaagca gaacttgttt attgcagctt 10620

ataatggtta caaataaagc aatagcatca caaatttcac aaataaagca tttttttcac 10680

tgcattctag ttgtggtttg tccaaactca tcaatgtatc ctatcatgtc tggatcgatc 10740

ctctacgccg gacgcatcgt ggccggcatc accggcgcca caggtgcggt tgctggcgcc 10800

tatatcgccg acatcaccga tggggaagat cgggctcgcc acttcgggct catgagcgct 10860

tgtttcggcg tgggtatggt ggcaggcccc gtggccgggg gactgttggg cgccatctcc 10920

ttgcatgcac cattccttgc ggcggcggtg ctcaacggcc tcaacctact actgggctgc 10980

ttcctaatgc aggagtcgca taagggagag cgtcgacctc gagggggggc ccggtacc 11038

## Description

### TECHNICAL FIELD

The present invention relates to a transgenic nonhuman animal, into which recombinant DNA comprising a heparin-binding EGF gene and a type 2 keratin gene promoter for regulating expression of the above gene has been introduced.

### BACKGROUND ART

The present inventors have previously focused attention on the fact that heparin-binding EGF is a main body of a receptor for toxin (diphtheria toxin) generated by *Corynebacterium diphtheriae,* so as to develop a method known as TRECK method (International Publication WO98/33899; Saito M, et. al., (2001) Nat. Biotechnology 19: 746-750). Namely, this method uses mouse cells having resistance to diphtheria toxin added from outside of the cells, which is 1,000 times or more higher than the same type of resistance of human cells, so as to specifically destroy only a specific mouse cell group. A summary of the method is as follows. First, a mouse cell- or tissue-specific promoter is allowed to bind to a diphtheria toxin receptor (DTR) that is specific for a human, so as to produce recombinant DNA. The produced recombinant DNA is then introduced into a mouse, so as to produce a transgenic (Tg) mouse. Because of the introduced promoter, the thus produced Tg mouse is able to express DTR specifically on the surface of a cell. Thus, if diphtheria toxin (DT) is administered to this Tg mouse, it becomes possible to specifically destroy a cell that expresses the above promoter at any given time. To date, this method has already been used to create a human disease model such as a model of hepatitis. Application of this method would enable creation of various types of models.

Atopic dermatitis is an allergic disease caused by an allergy reaction, and it is a chronic disease attended with itching. In recent years, this allergic disease tends to increase over the world. In particular, the incidence of this disease has significantly increased among young children. At the same time, there have been grave consequences such as an increase in the diseases, deterioration thereof, and expansion of the disease into old generations. Model mice have highly contributed to clarification of the cause of human atopic dermatitis or the development of a therapy therefor. NC mice are useful human atopic dermatitis model mice, which have been discovered in recent years. The number of the NC mice used has rapidly increased in the last ten years. However, since the conventional NC mice have been haired, it has been necessary to cut their hair for observation of the onset of dermatitis, which has been conducted as a basal experiment for clarification of the cause of atopic dermatitis or the development of a therapy therefor. Shaving hair from mice has required enormous efforts, and this shaving operation has needed human and temporal burden and high expenses. Moreover, there have also been many cases where stimulation by such hair shaving brings on the secondary onset of dermatitis. Thus, it has been extremely difficult to determine whether an initial stage of dermatitis observed in hair-shaved mice is the original atopic dermatitis or the secondary dermatitis.

### DISCLOSURE OF THE INVENTION

It is an object of the present invention to provide a transgenic nonhuman animal, which has acquired such a new phenotype as being hairless as a result of introduction of recombinant DNA comprising a heparin-binding EGF gene acting as a main body of a diphtheria receptor and a type 2 keratin gene promoter for regulating expression of the above gene into a nonhuman animal.

The present inventors have conducted intensive studies directed towards achieving the aforementioned object. The inventors have introduced recombinant DNA comprising a heparin-binding EGF gene and a type 2 keratin gene promoter for regulating expression of the above gene, used in the TRECK method, into a mouse, so as to produce a transgenic mouse. As a result, the inventors have succeeded in producing a mouse, which is congenitally hairless without administration of diphtheria toxin and wherein such a hairless state is inherited according to Mendel's law of inheritance, thereby completing the present invention.

That is to say, the present invention is as follows.
(1) A transgenic nonhuman animal, into which recombinant DNA comprising a heparin-binding EGF gene and a type 2 keratin gene promoter for regulating expression of the above-described gene has been introduced.
   The transgenic nonhuman animal of the present invention is characterized in that it is hairless, and is further characterized in that it develops dermatitis. Such dermatitis includes atopic dermatitis.
   The animal of the present invention is any one selected from the group consisting of a mouse, a rat, a rabbit, a guinea pig, a hamster, a dog, a cat, a swine, a sheep, and a goat. It is preferably a mouse.
(2) A method of producing a transgenic nonhuman animal, which is characterized in that it comprises introduction of recombinant DNA comprising a heparin-binding EGF gene and a type 2 keratin gene promoter for regulating expression of the above-described gene into a nonhuman animal. The transgenic nonhuman animal obtained by the method of the present invention is characterized in that it is hairless.
(3) A method of screening a therapeutic agent used for dermatitis, which is
characterized in that it comprises administration of a candidate substance to the transgenic nonhuman animal according to (1) above. Such dermatitis includes atopic dermatitis.

### BRIEF DESCRIPTION OF THE DRAWINGS

Figure 1 shows a portion of a vector used in production of the transgenic (Tg) mouse (NC-Krt2-6g-TRECK-Tg mouse) of the present invention.
Figure 2 is a view showing detection of the introduced gene by the PCR method.
Figure 3 is a photograph showing an approximately one-week-old transgenic mouse (Tg) and an approximately one-week-old wild-type (Wild) mouse.
Figure 4 includes photographs of an approximately 6-week-old transgenic mouse.
Figure 5 includes photographs of a dermatitis-developed transgenic mouse. The upper photograph shows inflammation of the skin, and the lower photograph shows a scratching behavior caused by itching.
Figure 6 shows the ratio between the cell surface antigens of bone marrow cells and those of spleen cells, which is obtained by applying a positive selection method using a flow cytometry to a transgenic mouse and a wild-type mouse.
Figure 7 shows the ratio of the cell surface antigens of peritoneal B cells, which is obtained by applying a positive selection method using a flow cytometry to a transgenic mouse and a wild-type mouse (left: B1 B220+CD5+; right: B2 B220+CD5-),
Figure 8 shows the ratio of the cell surface antigens of thymocytes, which is obtained by applying a positive selection method using a flow cytometry to a transgenic mouse and a wild-type mouse.

### BEST MODE FOR CARRYING OUT THE INVENTION

The present invention relates to a hairless transgenic nonhuman animal, into which recombinant DNA comprising a heparin-binding EGF gene and a type 2 keratin gene promoter for regulating expression of the above gene has been introduced.

### 1. Recombinant DNA

The present invention is characterized in that a hairless transgenic animal has been acquired by introduction of recombinant DNA comprising a heparin-binding EGF gene and a type 2 keratin gene promoter for regulating expression of the above gene into an animal. The recombinant DNA of the present invention will be described below.

### (1) Heparin-binding EGF gene

The heparin-binding EGF (HB-EGF; heparin binding-epidermal growth factor) gene of the present invention is a main body of a diphtheria toxin receptor. The above gene includes a membrane-bound type and a free type obtained by action of protease. In the present invention, a membrane-bound type is used. The nucleotide sequence of such a heparin-binding EGF gene is as shown in SEQ ID NO: 6.

In addition, the heparin-binding EGF gene of the present invention includes not only DNA having the nucleotide sequence as shown in SEQ ID NO: 6, but also DNA, which hybridizes with a sequence complementary to the nucleotide sequence as shown in SEQ ID NO: 6 under stringent conditions, and which encodes heparin-binding EGF.

Such a heparin-binding EGF gene can be obtained from a human cDNA library and genomic library according to a known hybridization method such as colony hybridization, plaque hybridization, or Southern blotting, using DNA having the nucleotide sequence as shown in SEQ ID NO: 6 or a fragment thereof as a probe. For the details of such methods, please refer to "Molecular Cloning, A Laboratory Manual 2nd ed." (Cold Spring Harbor Press (1989)).

Examples of stringent conditions applied in the aforementioned hybridization include conditions consisting of 1 x SSC to 2 x SSC, 0.1% to 0.5% SDS, and 42°C to 68°C. More specifically, pre-hybridization is carried out at a temperature between 60°C to 68°C for 30 minutes or longer, and hybridization is then carried out with a probe.

Thereafter, the reaction product is washed 4 to 6 times in 2 x SSC and 0.1% SDS at

room temperature for 5 to 15 minutes.

### (2) Type 2 keratin gene promoter

In the present invention, a type 2 keratin gene promoter can be used as a promoter that allows a diphtheria toxin receptor to express. The nucleotide sequence of such a type 2 keratin gene promoter is as shown in SEQ ID NO: 7.

The type 2 keratin gene promoter is associated with expression of a keratin2-6g protein. Such a keratin2-6g protein is one type of type 2 keratin, and it is specifically expressed in the inner root sheath of a hair root. Such a keratin2-6g protein is identical to that conventionally known as K2irs.

The type 2 keratin gene promoter is searched in the database of Mouse Genome Sequencing Consortium (Ensembl mouse assembly of http://www.ensembl.org/Mus-musculus/), and thus, the nucleotide sequence information thereof can be obtained. The nucleotide sequence thereof is as shown in SEQ ID NO: 7.

Moreover, the type 2 keratin gene promoter of the present invention includes not only DNA having the nucleotide sequence as shown in SEQ ID NO: 7, but also DNA, which hybridizes with a sequence complementary to the nucleotide sequence as shown in SEQ ID NO: 7 under stringent conditions, and which encodes a region having type 2 keratin gene promoter activity.

Such a type 2 keratin gene promoter can be obtained from a human genomic library according to a known hybridization method such as colony hybridization, plaque hybridization, or Southern blotting, using DNA having the nucleotide sequence as shown in SEQ ID NO: 7 or a fragment thereof as a probe. For the details of such methods, please refer to "Molecular Cloning, A Laboratory Manual 2nd ed." (Cold Spring Harbor Press (1989)).

Examples of stringent conditions applied in the aforementioned hybridization include conditions consisting of 1 x SSC to 2 x SSC, 0.1% to 0.5% SDS, and 42°C to 68°C. More specifically, pre-hybridization is carried out at a temperature between 60°C to 68°C for 30 minutes or longer, and hybridization is then carried out with a probe. Thereafter, the reaction product is washed 4 to 6 times in 2 x SSC and 0.1% SDS at room temperature for 5 to 15 minutes.

### (3) Regulation of gene expression

The recombinant DNA of the present invention is obtained by binding a type 2 keratin gene promoter that is a cell- or tissue-specific promoter to a heparin-binding EGF. gene that is a diphtheria toxin receptor specific for Primates. Because of the presence of such a type 2 keratin gene promoter, the heparin-binding EGF gene is specifically expressed on the surface of a cell.

The amino acid sequence that constitutes HB-EGF in the case of Primates such as a human or a monkey slightly differs from that in the case of a mouse or a rat. Binding affinity for diphtheria toxin obtained when such diphtheria toxin is incorporated into a cell by receptor-dependent endocytosis also differs between Primates such as a human or a monkey and a mouse or a rat. A mouse cell shows resistance to diphtheria toxin. This is because, since mouse HB-EGF has affinity for diphtheria toxin that is significantly lower than the case of a human receptor, its function as a diphtheria toxin receptor becomes extremely weak, and thus diphtheria toxin cannot come into a cell together with the aforementioned receptor. However, if human-derived HB-EGF (hHB-EGF) acting as a diphtheria toxin receptor is allowed to express in a mouse cell, because of the human-derived HB-EGF introduced into the mouse cell, diphtheria toxin can be incorporated into the cell just as with the case of a human. As a result, even such a mouse cell becomes sensitive to diphtheria toxin. Utilizing this property, a mouse wherein hHB-EGF has been allowed to express in a cell group acting as a target is produced, and diphtheria toxin is then administered to the mouse when damage is intended to be given to the cell. Thus, only the cell used as a target can be eliminated, or temporal damage can be given to the cell. This method is known as a TRECK (toxin receptor-mediated cell knockout) method. In general, if diphtheria toxin (DT) is administered to such a mouse into which recombinant DNA has been introduced, a cell in which a type 2 keratin gene promoter is allowed to express can be specifically destroyed at any given time. However, a mouse into which the recombinant DNA of the present invention has been introduced is congenitally hairless without administration of diphtheria toxin.

It has been known that EGF is closely associated with a hair formation cycle. Such a hair formation cycle consists of a hair cycle wherein an elongation step and a resting step are periodically repeated. Specifically, such a hair cycle consists of an anagen cycle, a catagen cycle, and a telogen cycle. The anagen cycle lasts from 2 to 6 years, and approximately 85% of the total amount of hair is in such an anagen cycle. The catagen cycle lasts from 1 to 2 weeks after the anagen cycle. During the catagen cycle, a hair follicle becomes shorter (approximately 1/6 of the ordinary length), and the lower part thereof is destroyed. A hair papilla is dissociated from the hair follicle, and it remains in the lower part. The telogen cycle follows the catagen cycle, and it lasts from 5 to 6 weeks. During this cycle, hair does not grow, and it still binds to the hair follicle. At the end of the telogen cycle, the hair follicle comes into the anagen cycle again, so that new hair can be formed. Thus, while a hair formation cycle is repeated, the telogen cycle is converted to the anagen cycle by activation of EGF. On the other hand, the anagen cycle is converted to the catagen cycle by inactivation of EGF.

### 2. Transgenic animal

The transgenic animal of the present invention is characterized in that it is hairless and also in that it spontaneously develops atopic dermatitis. A method of producing the transgenic animal of the present invention and the phenotype thereof will be described below.

### (1) Production method of transgenic animal

The transgenic animal (including the progenies thereof) of the present invention can be obtained by introducing the aforementioned recombinant DNA into the genome of a nonhuman mammal according to genetic recombination. The type of the nonhuman mammal used in the present invention is not particularly limited. Examples of such a nonhuman mammal include a mouse, a rat, a rabbit, a guinea pig, a hamster, a dog, a cat, a swine, a sheep, and a goat. In the present invention, a mouse is preferably used because of its handlability and reproductivity.

A transgenic animal can be produced by a standard method using a mouse, such as a microinjection method using a zygote or an introduction method using ES cells.

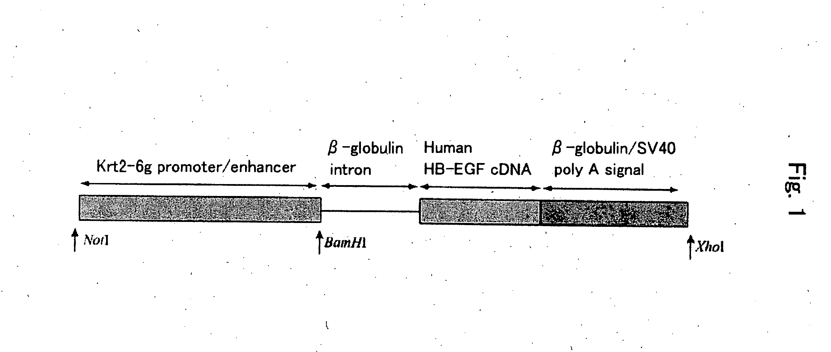

In the microinjection method, a gene to be introduced, which is used in microinjection for production of a transgenic animal, such as a cloning vector containing an expression cassette as shown in Figure 1 of the present invention, is produced. Subsequently, the expression cassette is cut out of the above expression vector by cleavage with restriction enzymes or the like. Thereafter, a purified DNA fragment is injected into the pronucleus of a fertilized mouse egg cell according to standard means (for example, Hogan, B. et al., (1994) Manipulating the Mouse Embryo 2nd edn., Cold Spring Harbor Laboratory Press, Cold Spring Harbor, New York). In order to identify a transgenic mouse, genomic DNA is prepared from ear-punched pieces or tail tissues. That is to say, ear-punched pieces or pieces of tail tissues are placed in a mixture of a PCR buffer, a nonionic surfactant, and proteinase K (50 mM KCl, 10 mM Tris-HCl, (pH8), 1.5 mM MgCl₂, 0.1% gelatin, 0.45% NP-40, 0.45% Tween-20, and 100 µg/ml proteinase K). The obtained mixture is incubated at 55°C overnight. After completion of the incubation, in order to deactivate proteinase K, the reaction product is incubated at 95°C for 15 minutes. The thus obtained solution is used as a DNA sample. In order to confirm the introduced gene, for example, the following primers are used to amplify the hHB-EGF gene region of the present invention:
5'- GGATCCTGAGAACTTCAGGGTGAGTTTG -3' (SEQ ID NO: 4); and
5'- CCAGATCTGCCTCTTGCAAGTCACG -3' (SEQ ID NO: 5).
Using the introduced hHB-EGF gene region as a template, a PCR reaction is carried out for amplification. For such a PCR reaction, TaKaRa Ex-Taq polymerase (Takara Bio Inc.) and a reaction solution included therewith are used. As a PCR reaction, after completion of a reaction at 94°C for 2 minutes, a cycle consisting of 98°C-10 seconds, 60°C-1 minute, and 72°C-3 minutes is repeated 34 times, followed by a reaction at 72°C for 4 minutes, so as to amplify a DNA fragment of interest.

In the introduction method using ES cells, the obtained recombinant DNA is introduced into ES cells. Such ES cells are derived from the inner cell mass of a zygote at the blastocyst stage. These cells can be cultured, while they remain in an undifferentiated state *in vitro.* As such ES cells, both the previously established cell line and a newly established cell line can be used. Examples of such a cell line used herein include a TT2 cell line, an AB-1 cell line, a J1 cell line, and an R1 cell line. The ES cell line used herein can be appropriately selected from among the above cell lines, depending on the purpose of an experiment or a method applied. For introduction of a gene into ES cells, methods such as calcium phosphate coprecipitation, electroporation, lipofection, retrovirus infection, agglutination, microinjection, or particle gun can be adopted. Among others, electroporation is preferable because it enables easy treatment of a large number of cells.

ES cells, into which a gene to be introduced has been incorporated, can be tested by the screening of chromosomal DNA separated and extracted from colonies obtained by culturing a single cell on feeder cells according to Southern hybridization or the PCR method. Otherwise, a vector that contains a drug resistance gene or a reporter gene may also be used for selection. Examples of such a drug resistance gene include a neomycin phosphotransferase II (nptII) gene and a hygromycin phosphotransferase (hpt) gene. Examples of such a reporter gene include a β-galactosidase (lacZ) gene and a chloramphenicol acetyl transferase (cat) gene.

ES cells, regarding which incorporation of a gene to be introduced has been confirmed, are returned to the embryo derived from a nonhuman mammal of the same species, so that the ES cells can be incorporated into a cell mass of the host embryo, thereby forming a chimeric embryo. The chimeric embryo is then transplanted to a host parent, so that it is allowed to develop and grow therein, thereby obtaining a chimeric transgenic animal. Thereafter, a chimeric animal is selected from among offsprings born from the host parent. (In the case of a mouse, offsprings are born after approximately 17 days.) An animal having a high contribution ratio of chimeric animals is highly likely to be a germ line. A possible chimeric animal is mated with a normal animal, so as to confirm that it is a germ-line chimeric animal. Thereafter, the thus obtained germ-line chimeric animal is mated with a normal female to obtain the F1 generation, so as to establish a mutant animal line.

The thus obtained chimeric transgenic animal is a heterozygote, which has the introduced gene on only one chromosome of each homologous pair. In order to obtain a homozygote, which has recombinant DNA on the two chromosomes of each homologous pair, two heterozygotes (a brother and a sister) having the introduced DNA on only one chromosome of each homologous pair, which are selected from among F1 animals, may be mated. Expression of the recombinant DNA can be confirmed by PCR.

Among the obtained transgenic animals, those exhibiting a hairless phenotype are subjected to natural crossing or external fertilization for passage, so that hairless transgenic animals (including the progenies thereof) can be produced.

### (2) Hairless animal

The term "hairless animal" is used herein to mean an animal, which has acquired a hairless phenotype, and which does not have hair on the entire body surface or a portion thereof, or has an extremely small amount of hair.

Conventionally, when an experimental animal has been subjected to an experiment such as a surgery, it has been necessary to shave hair around the surgical site. In such a case, there has been a risk of causing infectious disease. In addition, in the observation of the initial stage of dermatitis of a human atopic dermatitis model mouse as well, stimulation by such hair shaving may develop the secondary dermatitis, and it has made observation of the initial stage of atopic dermatitis more difficult. The hairless animal of the present invention is useful in that experiments can be carried out with the use of the above animal without such problems.

In the TRECK method, it is necessary to administer diphtheria toxin at the time of disruption of the introduced gene. However, since the hairless mouse of the present invention is congenitally hairless without administration of diphtheria toxin, it is excellent in terms of not only safety, but also a reduction in human and temporal burden and high expenses, which is caused by simple operations regarding the above hairless mouse.

### (3) Dermatitis animal

Dermatitis is a certain type of inflammation occurring on the skin, and it also includes asteatosis disease. In particular, the dermatitis animal of the present invention develops atopic dermatitis. Atopic dermatitis is an allergic disease, which is a chronic disease attended with itching. When the transgenic nonhuman animal of the present invention is exposed to an allergen, it spontaneously develops atopic dermatitis.

The inventors have previously used an NC mouse, a model animal that develops human atopic dermatitis, to specify a causative gene of the human atopic dermatitis. However, since such an NC mouse has been haired, it has been necessary to shave hair to observe the onset of dermatitis at the initial stage, as described above. Thus, it has been desired that a hairless mutant mouse having a novel genetic background as an NC mouse, which maintains the genetics of the conventional NC mouse with the only exception of being hairless, be produced. For example, the transgenic mouse of the present invention can be produced by isolating a Krt2-6 gene promoter that is considered to play an important role in formation of hair, and then introducing recombinant DNA that binds to diphtheria toxin receptor cDNA into the NC mouse. This transgenic mouse strain is hairless and is able to develop dermatitis without administration of diphtheria toxin. Such a phenotype as hairless is inherited according to Mendel's law of inheritance. As stated above, the genetics of this hairless mouse are advantageous in that its genetic background other than the hairless phenotype is identical to that of the existing NC mouse, but it is hairless. Thus, this hairless mouse is highly useful as a model mouse that develops dermatitis such as atopic dermatitis.

The onset of dermatitis can be confirmed by the appearance of any one or several symptoms selected from among erythema, papule, crust, and dander, in skin tissues on the whole body, or on any one or several sites selected from among face, head, neck, four limbs, pinna of ear, and back. There are also cases where the aforementioned symptoms are attended with epilation, bleeding, and crust formation. Moreover, in the case of mice, in addition to the aforementioned symptoms, a scratching behavior is observed in a large number of mice. These symptoms including minor changes observed at the initial stage are considered to be signs of the onset of dermatitis such as atopic dermatitis.

As stated above, the transgenic nonhuman animal of the present invention does not develop the secondary dermatitis caused by hair shaving, and thus it is useful as a model animal that develops atopic dermatitis and the like.

Furthermore, with regard to the transgenic nonhuman animal of the present invention, there are no abnormal findings specific for the transgenic nonhuman animal of the present invention in anatomical morphological observation of immunocompetent tissues such as thymus gland or spleen. For example, in the case of the transgenic mouse of the present invention, in order to compare its cell population that constitutes the immune system thereof with that of the existing NC mouse, the cell surface antigens of immunocompetent cells of the two types of mice can be compared and analyzed by a positive selection method using a flow cytometry. Among such immunocompetent cells, examples of thymus gland-derived cells include CD4 antigen- and CD8 antigen-positive cells, CD4 antigen-positive cells, CD8 antigen-positive cells, CD4 antigen- and CD8 antigen-negative cells, and CD4 antigen- and CD25 antigen-positive cells. Examples of splenic cells and bone marrow cells include CD3 antigen- and CD4 antigen-positive cells, CD3 antigen- and CD8 antigen-positive cells, and CD4 antigen- and CD25 antigen-positive cells. Examples of B cells include an IgM antigen and a CD 19 antigen. Examples of dendritic cells include CD11c antigen- and CD11b antigen-positive cells, B220 antigen-negative cells, CD11c antigen- and CD11b antigen-positive cells, B220 antigen-negative cells, and CD11c antigen-, CD11b antigen- and B220 antigen-positive cells. Examples of monocytes include CD11b antigen- and Gr-1 antigen-positive cells, CD11b antigen-positive and Gr-1 antigen-positive high expression cells, and CD11b antigen and Gr-1 antigen low expression cells. Examples of NK cells include NK1.1 antigen-positive and DX5 antigen positive expression cells. Examples of NKT cells include NK1.1 antigen-positive, DX5 antigen-positive, and CD3 antigen-positive expression cells. Examples of peritoneal B cells include B220 antigen-positive and CD5 antigen-positive B 1 cells, and B220 antigen-positive and CD5 antigen-negative B2 cells. Examples of peripheral blood lymphocytes include CD69 antigen-positive and CD80 antigen-positive cells.

When the transgenic nonhuman animal of the present invention is compared with the existing NC mouse in terms of cells associated with immunological functions by the aforementioned positive selection method involving positive selection of cell surface antigens, there is no deficiency in immunocompetent cells, or there is no statistically significant difference in the number of cells. Thus, it can be said that the transgenic nonhuman animal of the present invention has an immune system that is equivalent to that of the existing NC mouse.

### 3. Method of screening therapeutic agent for dermatitis

In the present invention, a candidate substance (a test substance) for agents used for the treatment of dermatitis such as atopic dermatitis is administered to a transgenic nonhuman animal that develops dermatitis such as atopic dermatitis, so as to screen a therapeutic agent for dermatitis such as atopic dermatitis. For example, a substance used as a candidate for a certain agent is allowed to come into contact with the transgenic nonhuman animal of the present invention or a portion thereof, and an indicator value having a correlation with disease as a target is then measured in the above nonhuman animal or a portion thereof with which the above candidate substance has been come into contact. Thereafter, the obtained indicator value is compared with that of a control. Based on the comparative results, it is confirmed whether or not the candidate substance is able to alleviate or eliminate the symptoms of dermatitis such as atopic dermatitis, so as to screen the candidate substance.

The term "a transgenic nonhuman animal or a portion thereof" is used to include both the whole body of a living animal, and the tissues or organs thereof. In the case of such tissues or organs, those excised from animals are also included. In the present invention, the skin is preferable.

Examples of a candidate substance include a peptide, a protein, a nonpeptide compound, a synthetic compound, a fermented product, a cell extract, a cell culture supernatant, a plant extract, a tissue extract of mammal (e.g. a mouse, a rat, a swine, a bovine, a sheep, a monkey, a human, etc.), and blood plasma. Such compounds may be either novel compounds or known compounds. These candidate substances may form salts. Examples of such salts of candidate substances include salts with physiologically acceptable acids (e.g. inorganic acids and organic acids, etc.) or with bases (e.g. metal salts, etc.). In particular, physiologically acceptable acid-added salts are preferable. Examples of such salts include salts with inorganic acids (e.g. hydrochloric acid, phosphoric acid, hydrobromic acid, sulfuric acid, etc.) or salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.).

Examples of a method of allowing a test animal to come into contact with a candidate substance include oral administration, intravenous injection, topical application, subcutaneous administration, intracutaneous administration, and intraperitoneal administration. Such a method can be appropriately selected, depending on the symptoms of a test animal, the properties of a candidate substance, etc. Of these, topical administration is preferable. In addition, the dosage of a candidate substance can be appropriately selected, depending on an administration method, the properties of a candidate substance, etc.

When a certain candidate substance had been administered, for example, if alleviation or elimination of the symptoms of dermatitis such as atopic dermatitis was confirmed; the used candidate substance could be selected as a therapeutic agent for dermatitis.

The present invention will be more specifically described in the following examples. However, these examples are not intended to limit the scope of the present invention.

### [Example 1] Construction of an expression vector

A base pair (SEQ ID NO: 7), which was located approximately 9 kb upstream of the start codon of a Kratin2-6g gene and which was assumed to include a promoter associated with expression of a Kratin2-6g protein and an enhancer sequence, was obtained from Ensemble database (http://www.ensembl.org/Mus_musculus/). The following primers were used to amplify the obtained base pair:
5'- AGCGGCCGCCAGATTATGAAAGGAACTCCTGCAAC -3' (SEQ ID NO: 1); and
5'- AGAGAGCACTGCGGAGCAACCACTGAAGC -3' (SEQ ID NO: 3).
Using genomic DNA derived from C57BL/6J(B6) as a template, the base pair was amplified by a PCR reaction. Such C57BL/6J(B6)-derived genomic DNA was prepared according to a standard method in the present laboratory, and it was then stored until use.

For such a PCR reaction, TaKaRa LA-Taq polymerase (Takara Bio Inc.) and a reaction solution included therewith were used. As a PCR reaction, after completion of a reaction at 94°C for 2 minutes, a cycle consisting of 98°C-10 seconds and 68°C-12 minutes was repeated 32 times, followed by a reaction at 72°C for 12 minutes.

Subsequently, a PCR reaction was carried out for amplification, using the thus amplified PCR product as a template, and also using the following primers:
5'- AGCGGCCGCCAGATTATGAAAGGAACTCCTGCAAC -3' (SEQ ID NO: 1); and
5'- TGGATCCGTTGGTAGAGGATGGAGTAAAGGTGC -3' (SEQ ID NO: 2).
For such a PCR reaction, TaKaRa LA-Taq polymerase (Takara Bio Inc.) and a reaction solution included therewith were used. As a PCR reaction, after completion of a reaction at 94°C for 2 minutes, a cycle consisting of 98°C-10 seconds and 68°C-12 minutes was repeated 32 times, followed by a reaction at 72°C for 12 minutes.

The thus amplified Kratin2-6g promoter and enhancer sequence were cleaved with NotI and BamHI, and the cleaved product was then incorporated into a human HB-EGF expression vector (Saito M, et. al., (2001) Nat. Biotechnology 19: 746-750).

A structure of the principal part of the expression vector mKr2-6gPro-hHB-EGF constructed by the aforementioned procedures is shown in Figure 1. The entire sequence of the vector is shown in SEQ ID NO: 8.

### [Example 2] Production of transgenic mouse

A cloning vector comprising the DNA fragment produced in Example 1 was obtained using QIAprep Spin Miniprep Kit (Operon). The cloning vector was then treated with the restriction enzymes NotI and XhoI (1 µg/unit) at 37°C. for 1 hour. Thereafter, the thus restriction enzyme-treated sample was subjected to agarose gel electrophoresis, so as to separate a DNA fragment to be used in microinjection. Subsequently, the DNA fragment was purified with QIAEX II (Operon). The purified DNA fragment was injected into the pronucleus of a fertilized NC mouse egg cell according to standard means (Hogan, B. et al., (1994) Manipulating the Mouse Embryo 2nd edn., Cold Spring Harbor Laboratory Press, Cold Spring Harbor, New York).

The mouse zygote, into which the gene to be introduced had been incorporated, was transplanted to a host parent, so that it was allowed to develop and grow therein, thereby obtaining a transgenic mouse.

In order to identify such a transgenic mouse, genomic DNA was prepared from a piece of tail tissue. That is to say, 0.1 g of a piece of tail tissue was placed in a mixture of a PCR buffer, a nonionic surfactant, and proteinase K (50 mM KCl, 10 mM Tris-HCl, (pH8), 1.5 mM MgCl₂, 0.1% gelatin, 0.45% NP-40, 0.45% Tween-20, and 100 µg/ml proteinase K). The obtained mixture was incubated at 55°C overnight. After completion of the incubation, in order to deactivate proteinase K, the reaction product was incubated at 95°C for 15 minutes. The thus obtained solution was used as a DNA sample.

In order to confirm the introduced gene, the following primers were used:
5'- GGATCCTGAGAACTTCAGGGTGAGTTTG -3' (SEQ ID NO: 4); and
5'- CCAGATCTGCCTCTTGCAAGTCACG -3' (SEQ ID NO: 5).
Using the introduced gene region as a template, a PCR reaction was carried out for amplification. For such a PCR reaction, TaKaRa Ex-Taq polymerase (Takara Bio Inc.) and a reaction solution included therewith were used. As a PCR reaction, after completion of a reaction at 94°C for 2 minutes, a cycle consisting of 98°C-10 seconds, 60°C-1 minute, and 72°C-3 minutes was repeated 34 times, followed by a reaction at 72°C for 4 minutes.

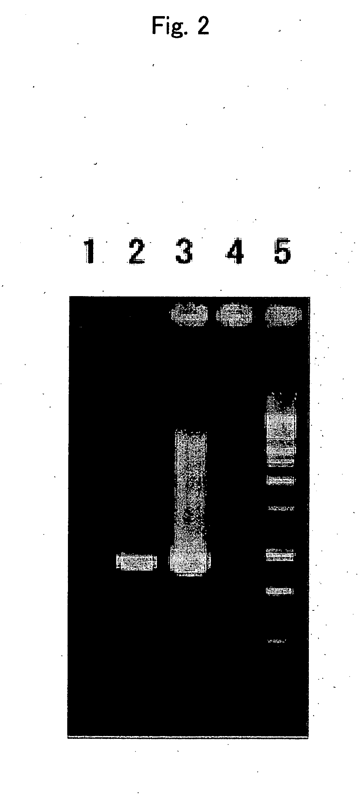

The results are shown in Figure 2. Figure 2 is a view showing detection of the introduced gene in mKr2-6gPro-hHB-EGF-transgenic (Tg) by the PCR method. Lane 1 indicates the results of a PCR reaction using the DNA of a non-introduced individual. Lane 2 indicates the results of a PCR reaction using the DNA of an introduced individual. Lane 3 indicates the results of a PCR reaction using a positive control expression vector mKr2-6gPro-hHB-EGF as a template. Lane 4 indicates the results of a PCR reaction using a negative control, namely, distilled water as a template. Lane 5 indicates a marker (250-10,000 bp).

As a result, it was confirmed that the mKr2-6gPro-hHB-EGF gene existed in the mouse genome in lane 2 (Figure 2). Hereafter, the mouse of lane 2 was used as mKr2-6gPro-hHB-EGF-Tg.

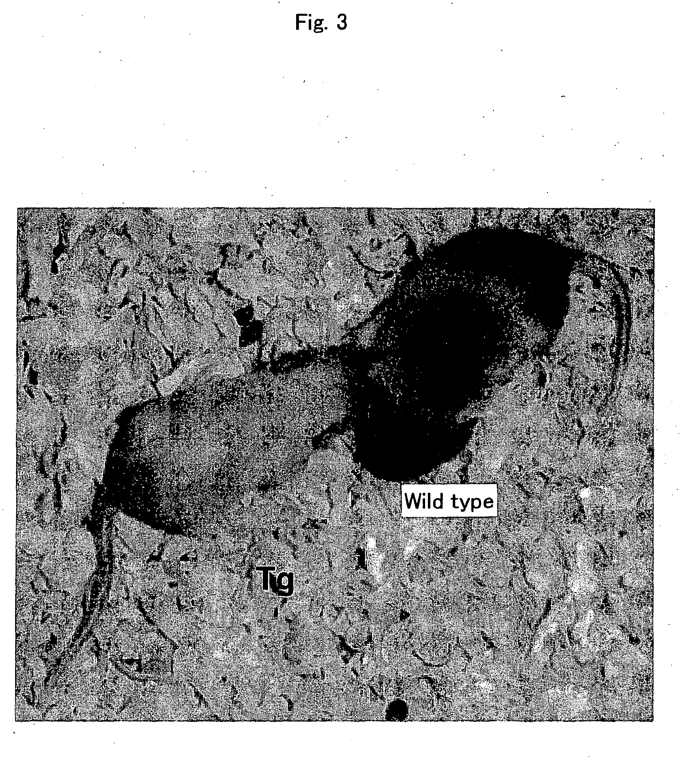

The thus produced mKr2-6gPro-hHB-EGF-Tg was hairless. This mouse was subjected to natural crossing with the existing NC mouse so as to obtain baby mice. Figure 3 shows the states of such baby mice. That is to say, a mouse having the mKr2-6gPro-hHB-EGF-introduced gene was hairless (left side; indicated as Tg), whereas a mouse that does not have such an mKr2-6gPro-hHB-EGF-introduced gene was haired (right side; indicated as wild type). The mKr2-6gPro-hHB-EGF-Tg of the present invention is a mouse, a hairless state of which can be inherited by natural crossing according to Mendel's law of inheritance.

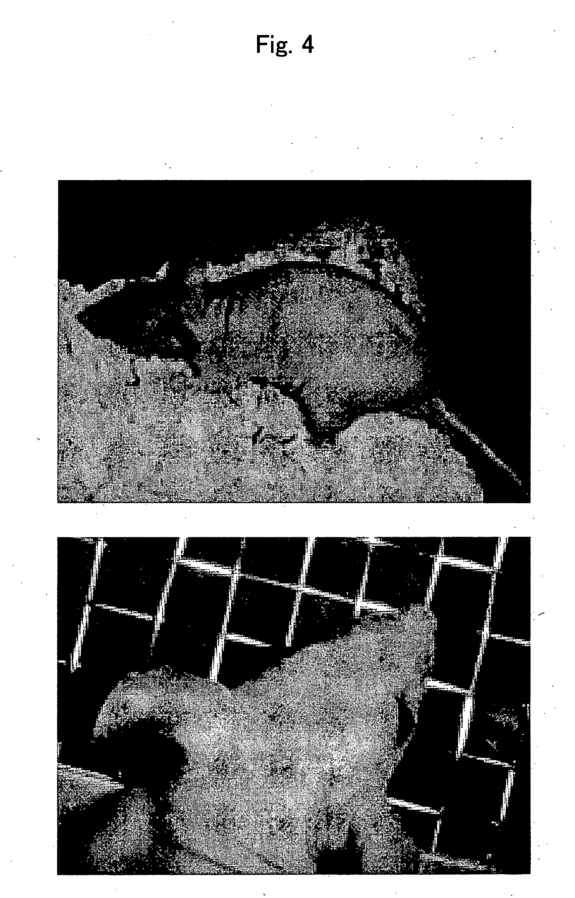

As stated above, baby mice obtained by natural crossing are continuously hairless. Figure 4 includes photographs of a 6-week-old mKr2-6gPro-hHB-EGF-Tg mouse, which has been hairless after birth.

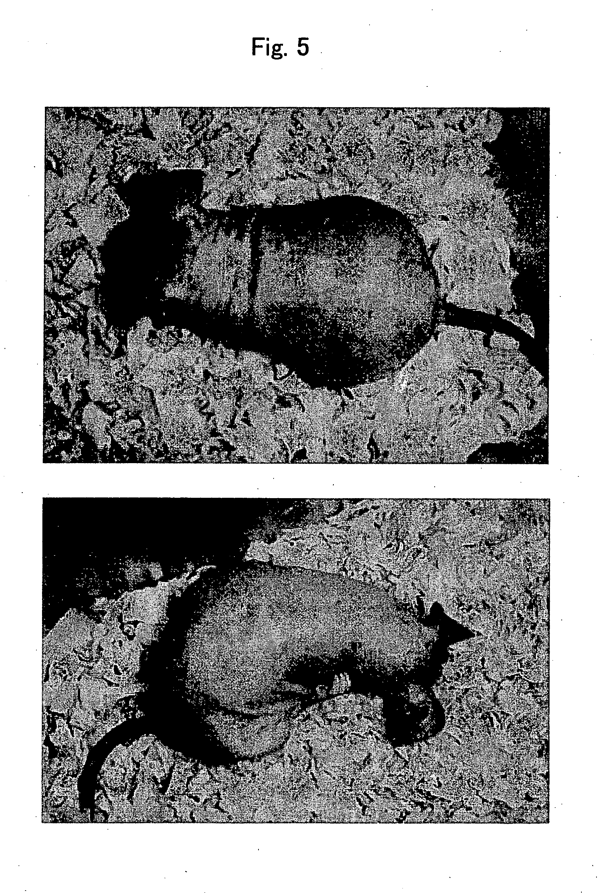

The mouse of the present invention, which has been bred in a conventional facility in which room temperature, humidity, and light and dark period are controlled, develops atopic dermatitis (Figure 5). The mouse as shown in Figure 5 is a 8-week-old mouse, which has been normally bred under the aforementioned conditions. The upper view of Figure 5 shows the onset of atopic dermatitis due to sensitization with an allergen existing in the breeding facility. The lower view of Figure 5 shows the scratching behavior of the mouse, which has developed atopic dermatitis due to itching.

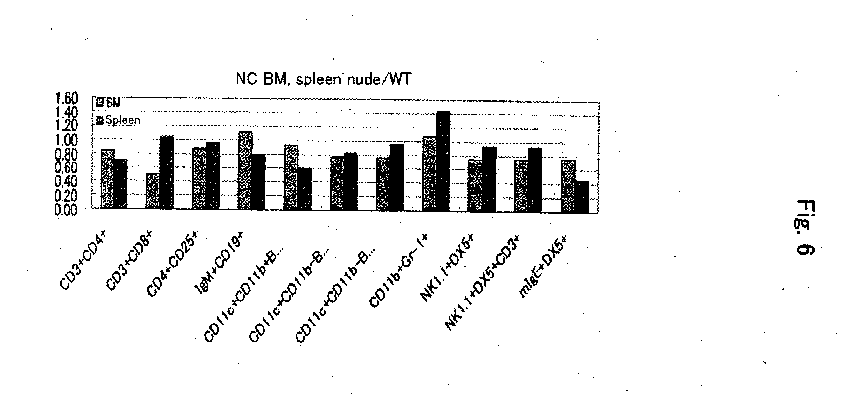

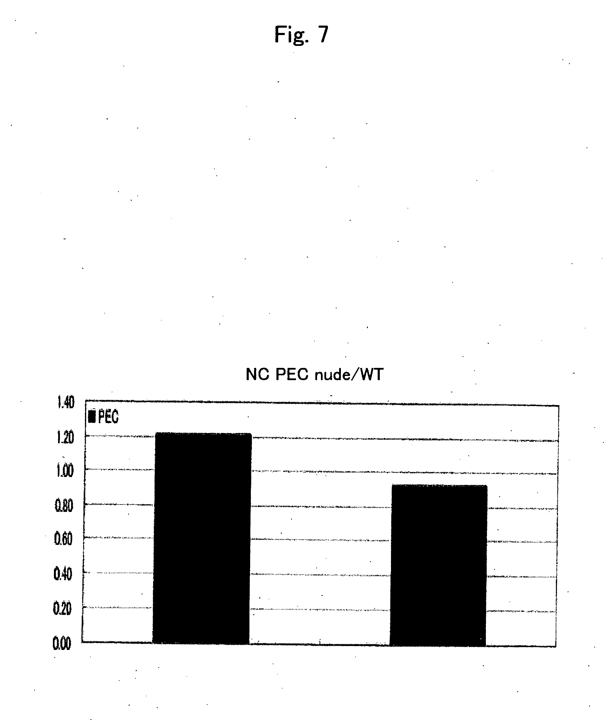

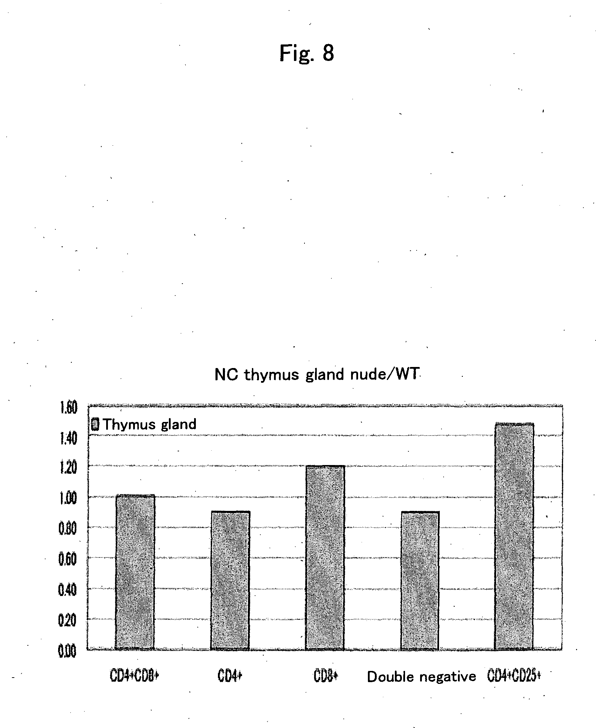

Moreover, with regard to the transgenic nonhuman animal of the present invention, there were no abnormal findings specific for the transgenic nonhuman animal of the present invention in anatomical morphological observation of immunocompetent tissues such as thymus gland or spleen. Furthermore, in order to compare the cell population of the transgenic mouse of the present invention that constitutes the immune system thereof with that of the existing NC mouse, the cell surface antigens of immunocompetent cells of the two types of mice were compared and analyzed by a positive selection method using a flow cytometry. The following immunocompetent cells were used. That is, examples of the used thymus gland-derived cells include CD4 antigen- and CD8 antigen-positive cells, CD4 antigen-positive cells, CD8 antigen-positive cells, CD4 antigen- and CD8 antigen-negative cells, and CD4 antigen- and CD25 antigen-positive cells. Examples of the used splenic cells and bone marrow cells include CD3 antigen- and CD4 antigen-positive cells, CD3 antigen- and CD8 antigen-positive cells, and CD4 antigen- and CD25 antigen-positive cells. Examples of the used B cells include an IgM antigen and a CD19 antigen. Examples of the used dendritic cells include CD11c antigen- and CD11b antigen-positive, B220 antigen-negative cells, CD11c antigen- and CD11b antigen-positive, B220 antigen-negative cells, and CD11c antigen-, CD11b antigen- and B220 antigen-positive cells. Examples of the used monocytes include CD11b antigen- and Gr-1 antigen-positive cells, CD11b antigen-positive and Gr-1 antigen-positive high expression cells, and CD11b antigen and Gr-1 antigen low expression cells. Examples of the used NK cells include NK1.1 antigen-positive and DX5 antigen positive expression cells. Examples of the used NKT cells include NK1.1 antigen-positive, DX5 antigen-positive, and CD3 antigen-positive expression cells. Examples of the used peritoneal B cells include B220 antigen-positive and CD5 antigen-positive B 1 cells, and B220 antigen-positive and CD5 antigen-negative B2 cells. Examples of the used peripheral blood lymphocytes include CD69 antigen-positive and CD80 antigen-positive cells. When the transgenic mouse of the present invention was compared with the existing NC mouse in terms of cells associated with immunological functions by the aforementioned positive selection method involving positive selection of cell surface antigens, there was no deficiency in immunocompetent cells, or there was no statistically significant difference in the number of cells. Thus, it was considered that the transgenic mouse of the present invention has an immune system that is equivalent to that of the existing NC mouse. The ratios of the cell surface antigens of bone marrow cells, spleen cells, peritoneal B cells, and thymocytes, are shown in Figures 6 to 8, as examples.

### Industrial Applicability

Since the transgenic animal of the present invention is hairless, it does not need hair shaving, which has previously been conducted on an ordinary NC mouse to observe the onset of atomic dermatitis at the initial stage. Accordingly, the secondary dermatitis induced by such hair shaving does not occur, and thus the transgenic animal of the present invention has an excellent effect of clearly determining the presence or absence of the onset of dermatitis such as atopic dermatitis at the initial stage.

In addition, the transgenic animal of the present invention is hairless, and thus the skin is not stimulated due to hair shaving. Thus, the above transgenic animal is useful for clarification of a mechanism to develop dermatitis such as atopic dermatitis or the development of a therapeutic agent for dermatitis such as atopic dermatitis. Moreover, such model animals can be used to open a new research area for clarification of detailed mechanism to develop dermatitis such as atopic dermatitis.

| Sequence Listing Free Text | |
|---|---|
| SEQ ID NO: 1 | Primer |
| SEQ ID NO: 2 | Primer |
| SEQ ID NO: 3 | Primer |
| SEQ ID NO: 4 | Primer |
| SEQ ID NO: 5 | Primer |
| SEQ ID NO: 8 | Vector |

## Claims

1. A transgenic nonhuman animal, into which recombinant DNA comprising a heparin-binding EGF gene and a type 2 keratin gene promoter for regulating expression of said gene has been introduced.

2. The transgenic nonhuman animal according to claim 1, which is **characterized in that** it is hairless.

3. The transgenic nonhuman animal according to claim 1 or 2, which is **characterized in that** it develops dermatitis.

4. The transgenic nonhuman animal according to claim 3, wherein the dermatitis is atopic dermatitis or atopic dermatitis-like disease.

5. The transgenic nonhuman animal according to any one of claims 1 to 4, wherein the animal is any one selected from the group consisting of a mouse, a rat, a rabbit, a guinea pig, a swine, a hamster, a dog, a cat, a sheep, and a goat.

6. The transgenic nonhuman animal according to any one of claims 1 to 4, wherein the animal is a mouse.

7. A method of producing a transgenic nonhuman animal, which is **characterized in that** it comprises introduction of recombinant DNA comprising a heparin-binding EGF gene and a type 2 keratin gene promoter for regulating expression of said gene into a nonhuman animal.

8. The method according to claim 7, which is **characterized in that** the transgenic nonhuman animal is hairless.

9. A method of screening a therapeutic agent used for dermatitis including atopic dermatitis, which is **characterized in that** it comprises administration of a candidate substance to the transgenic nonhuman animal according to any one of claims 1 to 6.

10. The method according to claim 9, wherein the dermatitis is atopic dermatitis or atopic dermatitis-like disease.
